# EUROPEAN PATENT SPECIFICATION

(11) **EP 1 651 155 B1**
(45) Date of publication and mention of the grant of the patent: **24.04.2013**
(21) Application number: 04780404.2
(22) Date of filing: 06.08.2004
(51) Int. Cl.: A61F 13/15, B26F 1/24, B26F 3/00, B29C 59/02

(54) **METHOD FOR MAKING AN APERTURED FILM**
VERFAHREN ZUR HERSTELLUNG EINES FILMS MIT ÖFFNUNGEN
PROCEDE POUR LA FABRICATION D'UN FILM PERFORE

(30) Priority: 07.08.2003 US 493207 P
(43) Date of publication of application: 03.05.2006
(60) Divisional application: 13159205.7
(73) Proprietor: The Procter & Gamble Company, Cincinnati, OH 45202 (US)
(72) Inventor: TURNER, Robert, Haines, Cincinnati, OH 45220 (US); BREIDENBACH, Vincent, Sean, Lebanon, OH 45036 (US); O'DONNELL, Hugh, Joseph, Cincinnati, OH 45215 (US); BENSON, Douglas, Herrin, West Harrison, IN 47060 (US)
(74) Representative: Kremer, Véronique Marie Joséphine
(86) International application number: PCT/US2004/025562
(87) International publication number: WO 2005/013874

(56) References cited:
- EP-A- 0 598 970
- WO-A-02/40250
- FR-A- 1 302 937
- US-B1- 6 551 436

## Description

### FIELD OF INVENTION

This invention relates to apertured webs. In particular, the present invention relates to apertured polymer films.

### BACKGROUND OF THE INVENTION

Apertured polymer films are known in the art. Such films find use in applications requiring film properties together with porosity. Such applications include ground covers, carpet backing signs and banners, as well as fluid pervious films for absorbent articles.

Current methods of aperturing film include hot punching, die punching, slitting and stretching, hydroforming and vacuum forming. Each of those processes has certain drawbacks, generally associated with a cost of manufacture. For example, any processes requiring heat input incurs energy costs associated with heat transfer.

Processes for aperturing films are described in, e.g., EP 598 970 A1 and FR 1302937. US 3097787 discloses an apertured film and a method for manufacturing the same by means of the apparatus described in US 2699208.

Accordingly, there is a need for a low cost apertured film. There is a need for a low cost method of making an apertured film, preferably without requiring heat or other energy-intensive process aids.

### SUMMARY OF THE INVENTION

The invention relates to a method of forming apertured films. The film is deformed to comprise a plurality of open-ended tent-like structures. In another embodiment not claimed the film can be deformed to comprise a plurality of slits. Optionally, the deformed film can be stretched to further open the tent-like structures or slits and increase the size of the aperture and flatten the film,

### BRIEF DESCRIPTION OF THE DRAWINGS

FIG. 1 is a photomicrograph of one embodiment of a web of the present invention compared to a millimeter scale.
FIG. 2 is a schematic representation of a process for making a web of the present invention.
FIG. 3 is a perspective view of a web after being processed by the deformation means of the apparatus for forming the web of the present invention.
FIG. 4 is a magnified portion of the web shown in FIG. 3.
FIG. 5 is a cross-sectional depiction of a portion of the web shown in FIG. 4.
FIG. 6 is a photomicrograph of another embodiment of a web of the present invention compared to a millimeter scale.
FIG. 7 is a perspective view of a portion of the apparatus for forming one embodiment of the film web of the present invention.
FIG. 8 is an enlarged perspective view of a portion of the apparatus for forming the web of the present invention.
FIG. 9 is a plot of tooth load versus strain.
FIG. 10 is a plot of tooth load versus strain.
FIG. 11 is a plot of force per ligament versus strain.
FIG. 12 is a plot of dart drop versus elongation from FIG. 11.
FIG. 13 is a plot of dart drop data for film formulations.
' FIG 14 is a plot of dart drop data for film formulations.
FIG. 15 is a photomicrograph of a film of the present invention.
FIG. 16 is a photomicrograph of a film of the present invention.
FIG. 17 is a photomicrograph of a film of the present invention.
FIG. 18 is a photomicrograph of a film of the present invention after the deformation step.
FIG. 19 is a photomicrograph of the film in FIG. 18 after the stretching step.
FIG. 20 is a photomicrograph of a film of the present invention after the deformation step.
FIG. 21 is a photomicrograph of the side view of the film in FIG. 20.
FIG. 22 is a photomicrograph of an enlarged view of the film in FIG. 20.
FIG. 23 is a photomicrograph of the film in FIG. 20 after the stretching step.

### DETAILED DESCRIPTION OF THE INVENTION

The present invention is directed toward a method of making low cost apertured films. The method of making the films has many possible variations depending upon the precursor film, equipment, processing conditions, and desired outcome. The preferred method of making the films is a very simple, high speed, durable process that can be run in a variety of environments. The robust process will utilize noncontacting aperturing rolls that intermesh but never touch. This reduces the wear on the system and provides less process break downs. Additionally, the process does not require any pattern registration, any rotational registration, or heating. The high speed process provides a variety of processing conditions and speeds that can utilize very different precursor films and produce wide variety of apertured films for very different end products.

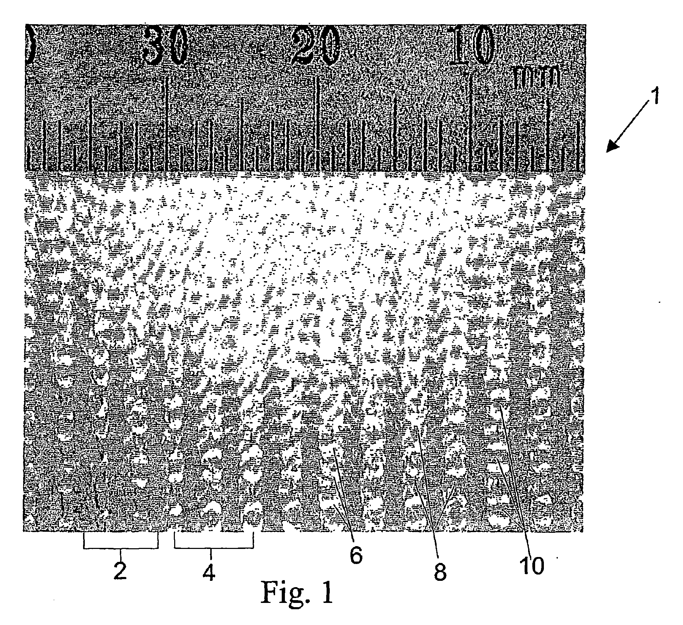

FIG. 1 is a photomicrograph showing one embodiment of a web 1 of the present invention compared to a millimeter scale. Web **1** is a generally flat, planar, two-dimensional polymer film having a machine direction (MD) and a cross-machine direction (CD) as is commonly known in the art of polymer film webs. A plurality of spaced apart, generally parallel first regions **2** are separated by a plurality of spaced apart, generally parallel second regions **4** in which openings define apertures **6.** First regions **2** and second regions **4** comprise the same material, preferably an extensible polyolefinic material such as polyethylene or polypropylene.

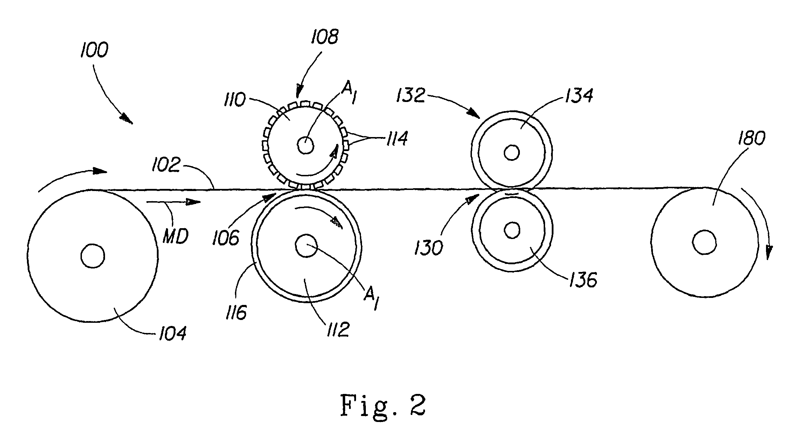

Web **1** can be formed by mechanical deformation and, if desired, incremental stretching of a generally planar, two-dimensional polymer film precursor web **102,** as described in more detail below with respect to FIG. 2. First regions **2** comprise material not significantly different in material properties from the precursor web **102,** while material properties of the second regions **4** differ from those of the precursor web **102,** at least with respect to molecular orientation and thickness. A transition region at the boundary between adjacent first and second regions comprises a complex blend of material properties.

In a preferred embodiment precursor web **102** is a polymer film web having a substantially random molecular orientation, that is, randomly oriented at least with respect to the MD and CD. By "substantially random molecular orientation" it is meant that, due to processing conditions during film extrusion, there may be a higher amount of long chain molecules oriented in the MD than the CD. This is normal and believed to be unavoidable in extruded film webs. After formation into an apertured web **1** of the present invention, however, second regions **4** exhibit a distinct CD molecular orientation. Molecular orientation can be determined by methods known in the art.

Openings in second regions **4** define apertures **6** which are spaced in a generally linearly oriented pattern in the MD direction. Apertures **6** provide for fluid communication from a first side to a second side of web **1.** As shown in FIG. 1, in one embodiment, the apertures **6** are generally oval, or kidney-shaped, and can be considered to be spaced apart pairs of apertures, each pair separated by a first, typically wide bridging portion or bridge **8** of second region **4.** Within each pair, each aperture can be separated by a second, typically narrow interaperture bridging portion or interaperture bridge **10.**

Referring to FIG. 2 there is shown an apparatus and method for making web 1 of the present invention. According to the invention, a precursor film web **102** is unwound from a supply roll **104** and travels in a direction indicated by the arrows associated therewith, i.e., the MD direction, as the supply roll **104** rotates in the direction indicated by the arrows associated therewith. The precursor film web **102** passes through nip **106** of the deformation means **108,** which comprises intermeshing rollers **110** and **112.** Before passing through deformation means **108,** the precursor film web **102** may be preheated or quenched. The deformation means **108** may be unheated or heated.

The deformation means **108** can comprise intermeshing rolls **110** and **112,** each rotating about an axis **A₁,** the axes **A₁** being parallel in the same plane. Roll **112** comprises a plurality of ridges **116** and corresponding grooves (not shown) which extend unbroken about the entire circumference of roll **112.** Roll **110** is similar to roll **112,** but rather than having ridges that extend unbroken about the entire circumference, roll **110** comprises a plurality of rows of circumferentially extending ridges that have been modified to be rows of circumferentially spaced teeth **114** that extend in spaced relationship about at least a portion of roll **110.** In operation, rolls **110** and **112** intermesh such that the teeth **114** of roll **110** extend into the grooves between ridges **116** of roll **112** and the ridges **116** of roll **112** extend into the grooves (not shown) of roll **110.** A roller arrangement suitable for use as deformation means **108** is shown in greater detail in published US Patent Application 2004/0131820A1.

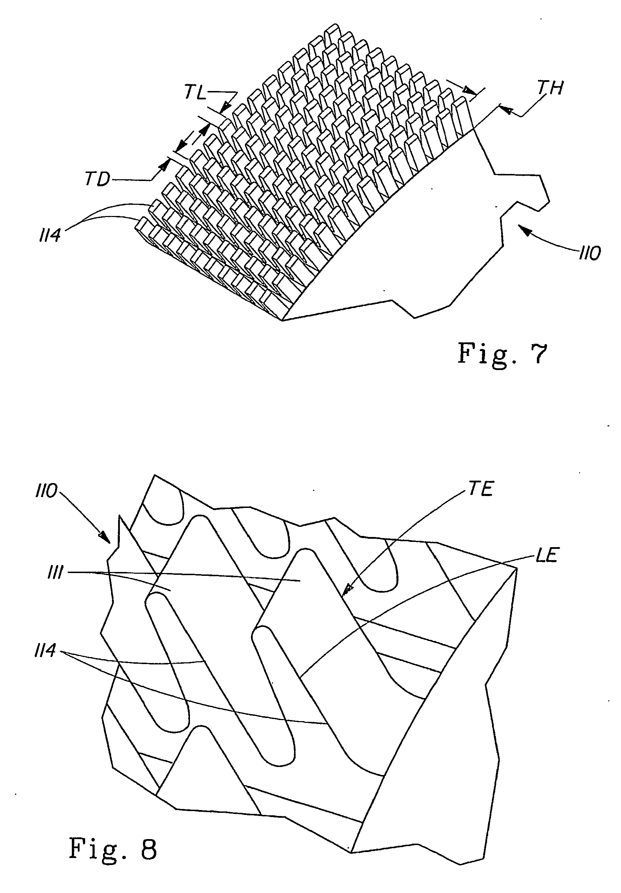

FIG. 7 illustrates a closer view of roll **110** which contains teeth **114.** This roll contains square shaped teeth **114.** As shown in FIG. 7, the tooth height **TH,** tooth distance **TD,** and tooth length **TL** can each be optimized to form the desired structure. As shown in FIG. 7, the tooth height **TH** is equal to the groove depth of the roll. In other embodiments, the tooth height **TH** can be less than the groove depth of the roll resulting in a much shorter tooth height. The shorter tooth will provide mechanical reinforcement of the tooth when the tooth length **TL** is very small resulting in fragile teeth.

An enlarged view of teeth **114** is shown in FIG. 7 is shown in FIG. 8. In this embodiment of roll **110,** teeth **114** have a uniform circumferential length **TL** which is measured from generally from the leading edge **LE** to the trailing edge **TE** at the tooth tip **111.** The teeth are uniformly spaced from one another circumferentially by a distance **TD.** The teeth **114** of roll **110** can have a length **TL** ranging from about 0.5mm to about 3 mm, a spacing **TD** from about 0.5 mm to about 3 mm, and a tooth height **TH** ranging from about 0.5 mm to about 10 mm. The pitch **P,** tooth-to-tooth or ridge-to-ridge spacing, between about 1 mm to about 2.54 mm. Depth of engagement, **E,** is the measure of the level of intermeshing of rolls **110** and **112** and is measured from the tip of the ridge to the tip of the tooth. Typical depth of engagement E can be from about 0.5 mm to about 5 mm. Each variable can be varied independently of each other to achieve a desired size, spacing, and area density of deformations.

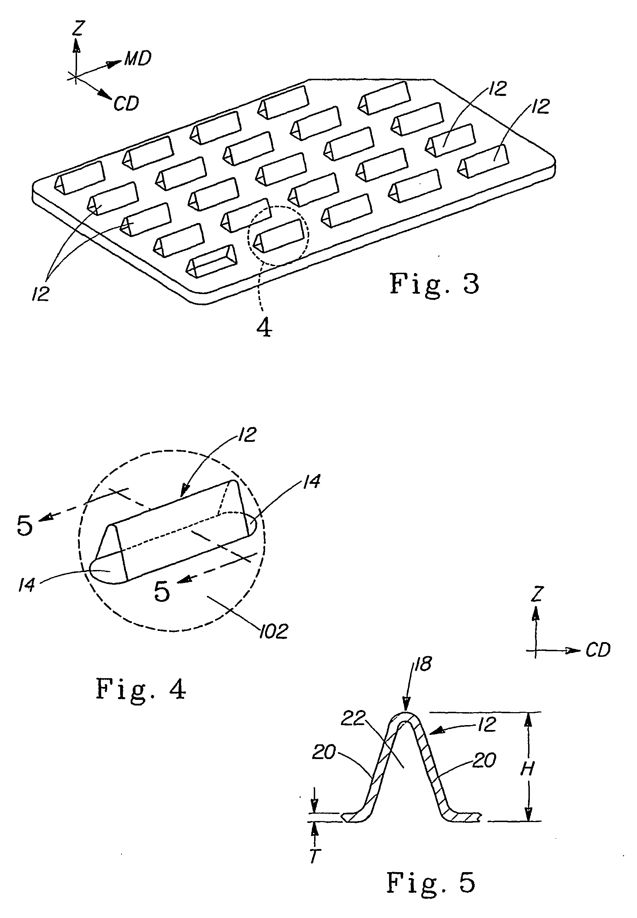

After leaving deformation means **108,** precursor film can be deformed to have discrete portions urged out of plane to form tent-like structures, as shown in FIG. 3, and referred to herein as deformations **12.** Deformations **12** correspond to the portions of precursor web **102** that teeth **114** of roll **110** pushed or punched through as precursor web **102** passed through nip **106.** In one embodiment, deformations **12** appear as shown in FIG. 4 as open-ended tent-like structures. As shown in FIG. 4, and in the cross-section of FIG. 5, deformation **12** can have sidewalls **20** that meet at a peak or ridge **18.** Deformations **12** can be open at both open ends **22.** In some embodiments, it has been noticed that open ends **22** can extend into the planar portion of precursor film web **102,** as denoted by **14** in FIG. 4. The tent-like structures may be open only at one end or not open at either end. The tent-like structure can have an aperture along the peak or ridge **18** where the sidewalls **20** meet. The sidewalls **20** may slightly overlap so that an aperture is not visible when the deformation **12** is formed but would be visible after a stretching step.

Partially formed precursor web **102** having deformations **12** has a rough texture, the level of roughness being proportional to the stiffness of the precursor web material, and the number and spacing of deformations **12.** In general, a web having deformations **12** can find use as an abrasive sheet, for example, for hard surface cleaning or sanding sheets.

After leaving deformation means **108,** the precursor film can be deformed to have slits. The slits correspond to the portions of the precursor web **102** that teeth **114** of roll **110** pushed or punched through as precursor web **102** passed through nip **106.** The precursor film can also be deformed to form other shapes such as bumps, ridges, or any protrusion into the Z-direction. In those formations, a slit may be described as on the top or tip of the deformation. Although the film and opening can have a variety of deformation shapes, the film will always be three dimensional, or moved into the Z-direction, after exiting the deformation means. The Z-direction is commonly understood in the nonwoven art to indicate an out-of-plane direction generally orthogonal to the MD-CD plane.

The teeth **114** of the roll **110** can be of various shapes and can have different degrees of sharpness. A square shaped tooth will generally produce a tent-like structure with two openings (one opening at each end) or possibly a slit along the peak of the tent-like structure. Therefore, one tooth can provide one or two openings. The corners of the tooth may have sharp corners. A square shaped tooth with rounded corners or an oval shaped tooth may be more likely to produce a slit. Similarly, a triangular tooth or pointed tooth may also produce a slit. In these cases, one tooth provides one opening. In other cases, a tooth with a small triangular point on top of a larger square tooth may form a tent-like structure with a slit in the middle which could produce three holes. Depending upon the speed of processing, the sharpness of the tooth, the dimensions of the tooth, and properties of the film such as the extensibility and basis weight, different structures will result. The shape of the tooth may be any suitable shape.

Referring again to FIG. 2, from deformation means **108** the film may continue to travel in the MD direction to another pair of intermeshing rollers, an incremental stretching means **132,** commonly referred to as ring rollers. Ring rollers **132** employ opposed rollers **134** and **136,** each having three-dimensional ridged surfaces which at least to a degree are complementary to one another. A ring roller apparatus suitable for use as incremental stretching means **132** is shown in greater detail in US Patent Number 5,628,097, filed September 29, 1995. The film can be heat treated just after the deformation means **108** and/or just after the stretching means **132.** The heat treatment process can be a heated nip, heated roll, hot oven, or other suitable heating process known to those skilled in the art.

The stretching means can be utilized to further open or enlarge the apertures. The stretching means can also be used to substantially flatten the three-dimensional film. After leaving the incremental stretching step of ring roller **132,** the apertured film web **1** of the present invention is fmished, and can be wound onto a take up roll 180 as shown in FIG. 2. Web **1** can be smoothed and flattened completely if necessary.

In another embodiment, a three-roll process could be utilized as compared to the four-roll process shown in FIG. 2. The number of rolls is counted as the rolls utilized in the deformation means **108** and the incremental stretching means **132.** As shown in FIG. 2, roll **110** containing teeth **114** would still be utilized with roll **112** containing groves. Another grooved roll, such as roll **134,** could be placed immediately adjacent to roll **112.** In this process, roll **112** is utilized as the roll holding the material for both the deformation means **108** and stretching means **132.** One advantage of this three-roll process is that there are no tracking issues. Tracking issues, where the film is no longer aligned or registered with the grooves or teeth of the rolls, may be more relevant when light weight films are used. Although sometimes desired, it is not required in the processes that the film is registered or tracked.

Referring back to FIG. 1, first regions **2** correspond to the portions of the precursor web **102** between the MD-oriented rows of tent-like structures, deformations **12,** as shown in FIG. 3. Second regions **4** correspond, at least in part, to what were the walls **20** of deformations **6,** as well as the portions of precursor web **102** between open ends of adjacent deformations **12,** prior to the incremental stretching step. Deformations **12** are stretched into a flattened, two-dimensional configuration by the incremental stretching of incremental stretching means **132,** but could, in theory, be flattened by any stretching means, such as by tentoring. Because second regions **4** represent highly deformed and stretched portions of web **1,** the thickness, T of the web, as indicated in cross-section in FIG. 5, is greater in first regions **2** than in second regions **4.** The molecular orientation of the polymeric web in second regions **4** is reoriented to be predominantly in the CD direction. The molecular orientation of the polymeric web in first regions **2** is not reoriented and remains substantially oriented as precursor web **102** which is generally in the MD direction. Apertures **6** correspond to the openings formed by the deformation rollers **108,** specifically openings **22** and **14.**

In one embodiment, after deforming web **102** through deformation means to form deformations **12,** for example, after passing through toothed roller arrangement **108,** the tent-like deformations **12** have a height, **H,** measured in the Z-direction as shown in FIG. 5, of at least 3 times the thickness **T** of the precursor film web **102.** In another embodiment, **H** can be greater than about 10, 20, 50, 100, or 150 times the thickness of web **102.**

In one embodiment, each aperture **6** has an area of at least about 0.1 square mm. The row-to-row spacing of first regions **2** can be from 1-10 mm, preferably 1-5 mm, and most preferably 2-3 mm. The teeth **116** of the roll **110** can be patterned. A pattern is created by either removing certain teeth or arranging teeth in a pattern. A pattern can also be created by altering roll **112** so that the teeth **116** of roll **110** do not deform the web **102.**

In one embodiment, web **102** can be stretched in the MD, either before or after the stretching step, for example, before or after stretching means **132.** MD stretching can be achieved by means known in the art, such as by use of S-wrapped rolls or by winding under high tension. Such drawing in the MD can result in larger apertures.

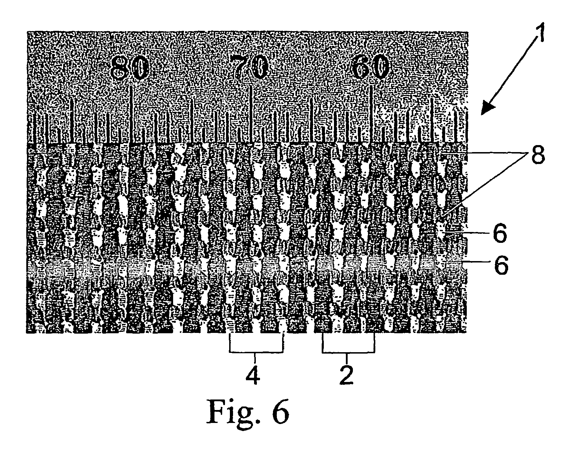

In another embodiment, as shown in FIG. 6, apertures **6** can be generally rectangular shaped. The web **1** shown in FIG. 6 was produced from a polypropylene film having a thickness of about 130-150 microns. During the process of going through the rollers of the deformation means **108,** precursor web **102** was pierced by teeth **114** to form slits separating slightly raised flaps instead of the tent-like structures shown in FIG. 3. Once stretched by stretching means, such as incremental stretching means **132,** the slits open into apertures, which can, as shown in FIG. 6, be generally rectangular in shape.

The number, spacing, and size of apertures 6 can be varied by changing the number, spacing, and size of teeth **114** and making corresponding dimensional changes as necessary to roll **110** and/or roll **112.** This variation, together with the variation possible in precursor webs **102** and line speeds, permits many varied webs **1** to be made for many purposes.

Polymer film precursor webs **102** can be any known polymer film webs having sufficient elongation, extensibility, or elasticity properties as desired. Preferably, the extensibility of the web will be greater than about 200%. To form apertures from a volcano-like structure, the extensibility of the web can be less than 200%. It may be desired that the film is not highly elastic or extensible as the apertures may close or shrink in size after processing. The properties of the film and processing condition will vary for each application. The basis weight of the film can be any suitable basis weight such the film does not shred during processing. Preferred basis weights are typically greater than about 40 gsm. Suitable basis weights are from about 20 gsm to about 200 gsm, preferably from about 40 gsm to about 100 gsm. The thickness of the film is typically less than about 40 mils, preferably from about 0.5 to about 30 mils and more preferably from about 1 to about 20 mils.

Polymeric materials suitable for use include polyethylene, polypropylene, polyesters, including PET polyester, polyvinyl chloride, and nylon, including nylon 6, nylon 6,6, and amorphous nylon. Other polymeric materials or combinations of materials having extensibility are also suitable. As used herein, the term "polymer" generally includes, but is not limited to, homopolymers, copolymers, such as for example, block, graft, random and alternating copolymers, terpolymers, etc., and blends and modifications thereof. In addition, unless otherwise specifically limited, the term "polymer" includes all possible geometric configurations of the material. The configurations include, but are not limited to, isotactic, atactic, syndiotactic, and random symmetries. The polymer web can be a laminate of two or more webs and can comprise coextruded layers of one web.

There are unlimited numbers of products made of an apertured web. Some examples include web **1** made from a high basis weight film can be useful as a ground cover, patio liner, or other agricultural films, such as for weed blocking. High basis weight films can also be utilized as a carpet backing, mechanical reinforcing scrim, or breathable house wrap. A web **1** made from a relatively low basis weight polymer film web could be used as a porous film barrier in disposable absorbent articles, such as a topsheet for a feminine protection pad. Other uses can include face masks, sunshades, or other articles desiring a breathable film. A stiffer or stronger apertured film material can be used for scrubbing applications such as scrubbing the floor or other hard surface cleaning. It could also be used as a pedicure product. The apertured films can be used to help control delivery of materials such as providing controlled release, encapsulation, or permeation of liquids. The apertured films can help to provide opacity to products. The apertured films may also be corrugated to help add strength or additional texture to the film. The corrugation can occur from the ring rolls used in a stretching process.

To aid in the selection of suitable film materials and predict the aperturing behavior, a ring roll simulation press can be utilized. The ring roll simulation press is utilized to determine the strain at which apertures **6** form and the strain at which interaperture bridges **8,** if present, break. These measurements can then be used to correlate with corresponding features in the load versus strain data. This enables suitable process setting to be selected to form the desired structures for the film material. More detailed information on the ring rolling simulation press is found in published application WO 2004/050341A1.

The roll simulation testing apparatus uses flat plates with intermeshing grooves and teeth machined into their surfaces. The geometry and dimensions of the grooves and teeth of the flat plate match those of the grooves and teeth on the rolls to be simulated. There is a stationary plate and a plate attached to a carriage. The carriage moves along an axis normal to the surface of the stationary plate. The plates are aligned parallel to each other so that when the carriage moves to bring the plates together, the grooves of the moving plate intermesh with the teeth of the stationary plate, just as the grooves of the toothed roll intermesh with the grooved roll in the nip **106** of rolls **110** and **112** in FIG. 2. Position detectors on the carriage are used to record the moving plate's position relative to the stationary plate. When the moving plate's position is intermeshed with a sample mounted on the patterned plate, the measure of the plate's position is directly used to determine the strain on the sample. Load sensors on the stationary plate record the load imposed by the moving plate and transmitted through the specimen mounted on the stationary plate. In order to acquire the load-versus-force data for the processing event, one mounts a sample in the press on the stationary plate, activates the loading cycle, and as the moving plate strains the film mounted on the stationary plate, a computer acquires the strain and associated load values for storage in a computer data file.

It is preferred to cycle the test at various maximum strains in order to examine what effects the various strains have on the sample. Using this method, one can determine if and at what strain the apertures **6** form, if and at what strain the interaperture bridges **10** break, and correlate these events with their corresponding features in the load versus strain data. Interaperture bridges **10** may or may not be formed depending upon the shape of the tooth, properties of the material, and settings for the process.

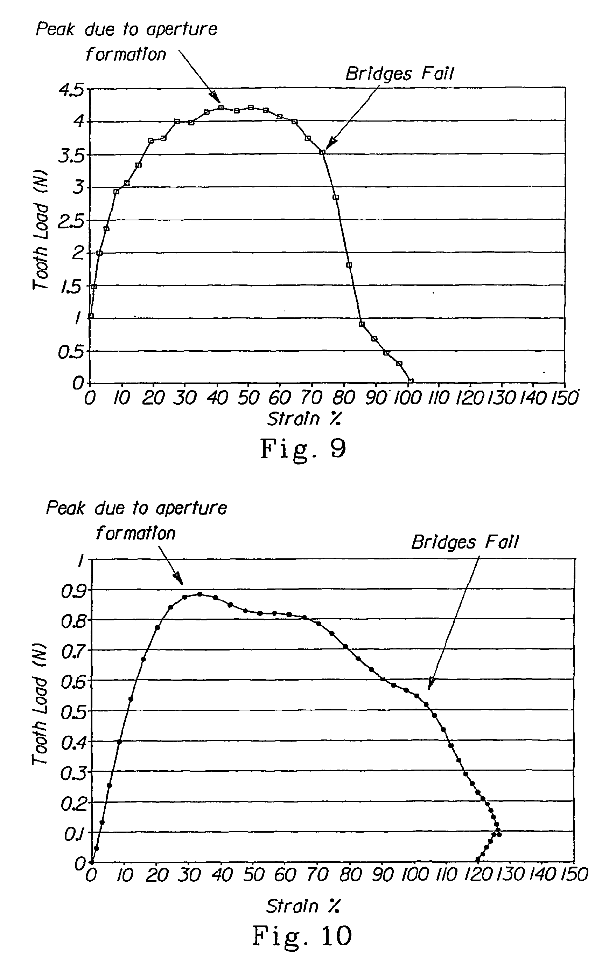

Data from the ring roll simulation press is shown in FIG. 9. FIG. 9 shows a plot of tooth load versus strain for a test run with a 0.152 mm (about 0.006 inch) thick polyethylene film produced by Sunbelt Plastics, Monroe, LA. The simulated line speed was 145 meters per minute. The data show that during the aperture formation event in the nip **106** of rolls **110** and **112** shown in FIG. 2, apertures formed just after straining beyond the maximum load response of the film, approximately 45% strain. Upon further straining, the interaperture bridges were stretched and even further strain resulted in failure of the bridges with full penetration of the tooth through the film. The failure of the bridges, approximately 75% strain, was accompanied by a sharp reduction in tooth force with increasing strain. Overall, the bridges were stretched from the point at which apertures formed, approximately 45% strain, to 75% strain before failing. The magnitude of the difference in strain between bridge failure and aperture formation, 30% in this example, was indicative of the processing range for the film to form stretched bridges.

FIG. 10 shows a plot of tooth load versus strain for a test run with a 0.025mm (about 0.001 inch) thick low-density polyethylene film made by Tredegar Film Products, Richmond, VA. The simulated line speed was 145 meters per minute. The data indicate that during aperture formation in the nip **106** of rolls **110** and **112** shown in FIG. 2, apertures formed just after straining beyond the maximum load response of the film, approximately 30% strain. Upon further straining, the interaperture bridges were stretched. However, unlike the case of the thicker film used to generate FIG. 9, the interaperture bridges of this film were able to withstand strain to much higher levels, approximately from 30% to 100% strain versus 45% to 75% for FIG. 9, before failing. The processing range for this film to form stretched bridges was 70%.

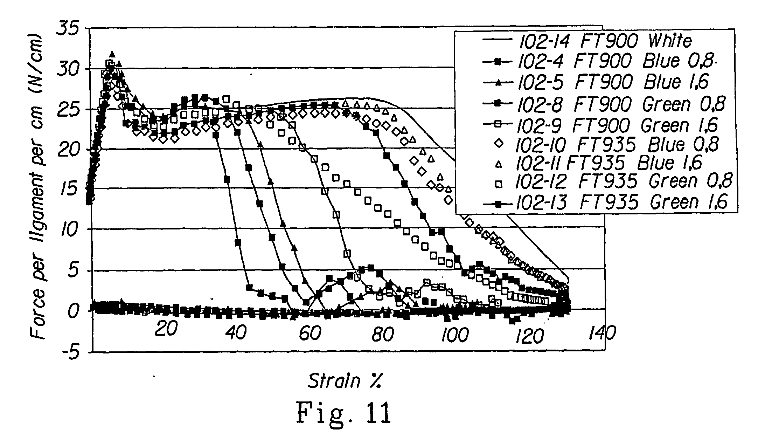

FIG. 11 shows a plot of load versus strain for a test run with a series of 0.1 mm (about 0.004 inch) thick polypropylene films produced by FilmTech in Allentown, PA. The films are formulated and sold as either FT900 or FT935. Each of these formulations is colored with either 0.8 or 1.6% of white, green, or blue colorant. The data show that during the deformation between the grooves of the plates, the force per ligament (the total force divided by the number of grooves that actively deform the web) rapidly increases within the first 10% strain and then reaches a plateau at about 25 Newtons/centimeter (N/cm). Upon further straining, the force per ligament eventually decreases and many films may shred into pieces at high strains. A primary means to characterize the ability of films to deform without shredding in the process is obtained from FIG. 11. The characterization is based on the strain reached after the force decays to 50% of the plateau force per ligament (i.e. strain at 50% decay). For these films, the strain at 50% decay occurs at 12.5 N/cm.

The ability of a film to deform at high strain rates is believed to be related to the impact resistance of the film. Not being bound by theory, it is believed that the strain at 50% decay is related to the impact properties as measured by the dart drop method (ASTM D1709, Method A). This method is an industry standard method and does not require a highly specialized instrument.

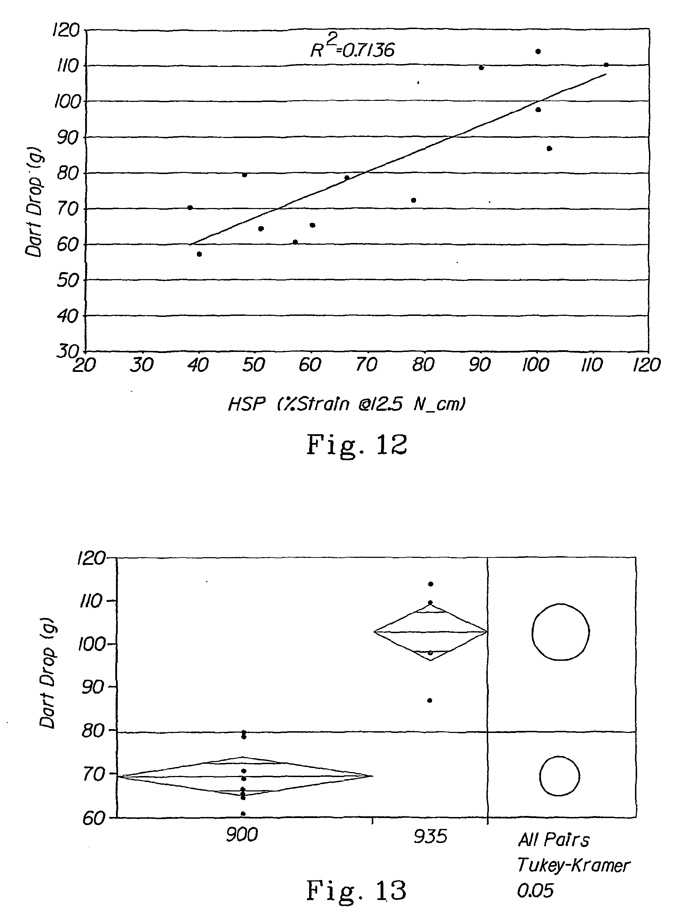

FIG. 12 shows the relationship between dart drop and strain at 50% decay for the films presented in FIG. 11. There is a strong relationship between these two methods indicating that impact resistant films are more able to extend at high strain rates. Based on creating apertured films using the processes of the present invention, a dart drop of 85 g or greater is most preferred for high speed production processing (typically greater than 300 fpm). Films having lower dart drop may be suitable when created at lower production speeds.

Not being bound by theory, it is believed that aperturing behavior changes as the impact resistance of a film increases. Starting from low impact resistance films and progressing to high impact resistant films, it is believed that the film may transition in the following progression when processed on a square, sharp edged tooth: i) gross film fracture leading to film breakage; ii) localized brittle fracture surrounding the entire tooth leading to a single jagged aperture per tooth; iii) brittle fracture surrounding the two corners of the tooth leading to two jagged apertures per tooth, iv) ductile failure surrounding the two corners of the tooth leading to two smoother apertures per tooth; v) ductile drawing leading to no apertures. The behavior of the film is dependent on factors such as material composition, morphology, and deformation rate. It is also highly dependent upon tooth geometry and size and other processing conditions.

The upper limit of dart drop strength for a film is only limited by the ability to aperture the film. Higher dart drops are typically obtained by inclusion of impact modifying polymers. For polypropylene films, suitable impact modifying resins are typically blended into the polypropylene resin during extrusion to create a second impact resistant phase.

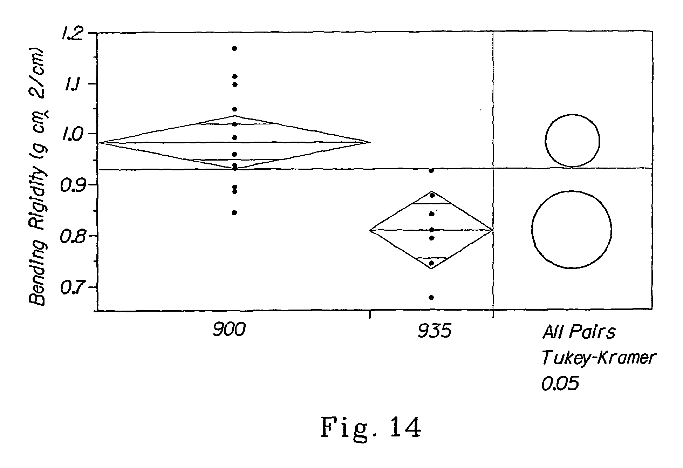

FIG. 13 shows that formulated FT900 has lower dart than formulated FT935. Contrarily, FIG. 14 shows that FT900 has higher modulus than FT935. The data are displayed using JMP statistical graphs from SAS Institute Inc., Cary, NC. Increased impact modifying resin content typically leads to lower polypropylene film modulus and increased impact resistance. For film uses where higher modulus is desired, a limited amount of impact modifiers is desired. Hence, higher modulus but lower dart drop film FT900 may be desired for applications such as soft abrasive structures where higher modulus is desired. This apertured film can be made at low to moderate production speeds.

### EXAMPLES

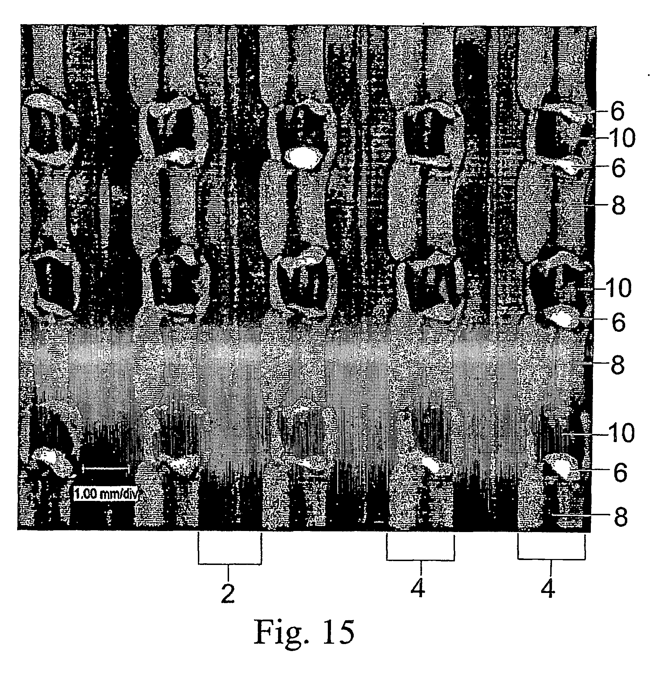

The precursor film used to make the apertured film shown in FIG. 15 was a 0.152 mm (about 0.006 inch) thick black polyethylene film produced by Sunbelt Plastics, Monroe, LA. The apertured film shown in FIG. 15 was apertured using the nip **106** of rolls **110** and **112** having a depth of engagement **E** of about 1.65 mm (about 0.065 inch), a pitch **P** of about 1.5 mm (about 0.060 inch), a tooth height **TH**, of about 3.7 mm (about 0.145 inch), a tooth distance of **TD** of 1.6 mm (abut 0.063 inch), and a tooth length of **TL** of about 1.25 mm (about 0.050 inch). During the same process and after aperturing, the apertured film was then stretched using a ring roll at a depth of engagement **E** of about 1.5 mm (about 0.060 inch) and a pitch **P** of about 1.5 mm (about 0.060 inch). The web was run at a line speed of about 15 meters/minute (about 50 feet per minute). The photograph in FIG. 15 was taken at 25X magnification with incident illumination and a white background. As can be seen in FIG. 15, the first region **2** remains substantially unchanged. Second region **4** can be observed to have thinned film and apertures **6.** The interaperture bridges **10** remain intact. The bridges **8** between the apertures are also shown.

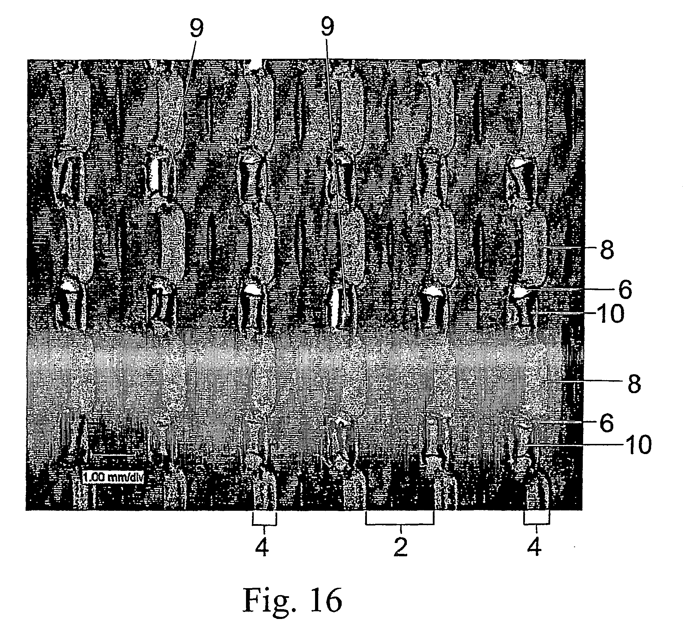

The apertured film shown in FIG. 16 was made from the same black polyethylene precursor film and was made in a similar fashion as the apertured film shown in FIG. 15, except the nip **106** of rolls **110** and **112** had a depth of engagement **E** of about 1.1 mm (about 0.045 inch) and the ring roll had a depth of engagement of 0.9 mm (about 0.035 inch). The web was run at a line speed of about 150 meters/minute (about 500 feet per minute). This apertured film has suitable properties for use as an agricultural weed blocking layer. Its opacity blocks sunlight and its apertures block plant growth but permit the passage of water and air to soil underneath the film. The photograph in FIG. 16 was taken at 25X magnification with incident illumination and a white background. As can be seen in FIG. 16, the first region **2** remains substantially unchanged. The slightly deformed shape near the center of the first regions 2 is from the ring roll utilized in the stretching step. Second region **4** can be observed to have thinner film and apertures **6.** Some of the interaperture bridges **10** remain intact while others are broken. The broken interaperture bridges **9** are due to the high speed processing and increased strain rate on the film. The bridges **8** between the apertures are also shown.

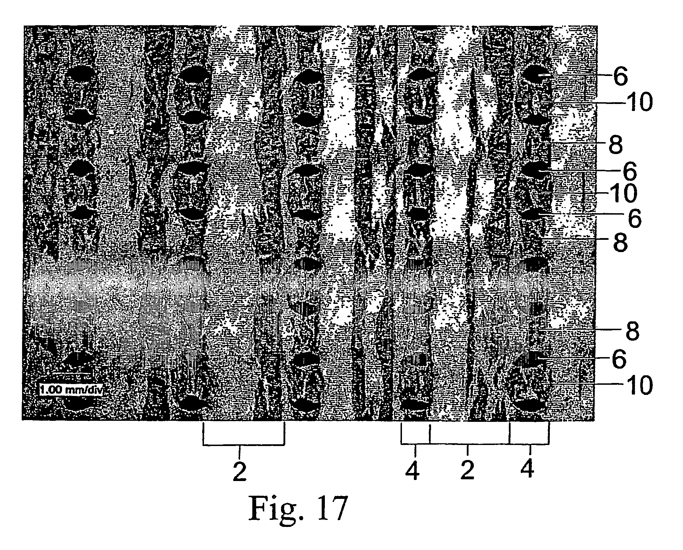

The precursor film used to make the apertured film shown in FIG. 17 was a 0.025mm (about 0.001 inch) thick white low-density polyethylene film made by Tredegar Film Products, Richmond, VA. The apertured film shown in FIG. 17 was apertured using the nip **106** of rolls **110** and **112** having a depth of engagement **E** of about 1.4 mm (about 0.055 inch), a pitch **P** of about 1.5 mm (about 0.060 inch), a tooth height **TH**, of about 3.7 mm (about 0.145 inch), a tooth distance of **TD** of 1.6 mm (abut 0.063 inch), and a tooth length of **TL** of about 1.25 mm (about 0.050 inch). During the same process and after aperturing, the apertured film was then stretched using a ring roll at a depth of engagement **E** of about 1.5 mm (about 0.060 inch) and a pitch **P** of about 1.5 mm (about 0.060 inch). The web was run at a line speed of about 275 meters/minute (about 900 feet per minute). This apertured film has suitable properties for use as a topsheet in feminine hygiene products that provides for superior fluid acquisition and superior rewet properties (i.e., reduced fluid movement back to the surface of the topsheet). The photograph in FIG. 17 was taken at 25X magnification with incident illumination and a black background. As can be seen in FIG. 17, part of the first region **2** remains substantially unchanged. The slightly thinned areas the first regions **2** is from the ring roll utilized in the stretching step not being perfectly registered with the roll from the deformation step. Second region **4** can be observed to have thinner film and apertures **6.** The interaperture bridges **10** remain intact. The very light stripe down the center of the interaperture bridges **10** is where a tooth held the film during processing. The bridges **8** between the apertures are also shown.

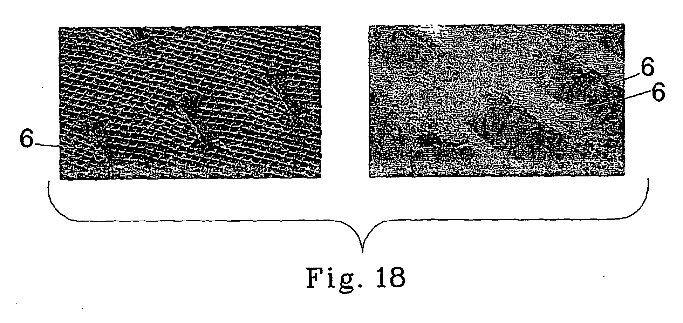

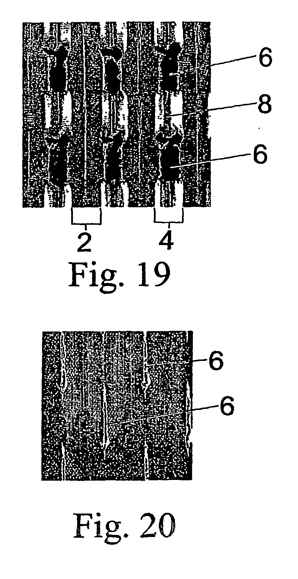

These films can be used to create fractured or apertured films using a one (deformation means) or two-step process (deformation and stretching means). FIG. 18 shows the film deformations with slits after processing with the deformation means and FIG. 19 shows the film deformation and apertures after the second step of processing. The first step in the process consists of using the teeth in the deformation means to create brittle fractures or slit apertures across the entire length of the tooth. FIG. 18 shows both sides of a FT900 film after processing at 750 fpm. The photograph on the left shows a micro taffeta emboss pattern on the embossed side of the film and the photograph on the right shows the smooth side of the film. Both photographs illustrate a tent-like deformation with a slit or fracture along the peak. The sidewalls of the tent-like deformation may be overlapping. This film can be used in this form to create an abrasive film consisting of protrusions and/or apertures. The open area of these films is typically between 0 and 5%.

If the second step is desired, a stretching means can be used to expand the fractured or apertured film to create open apertures. The second step consists of engaging the film between a set of grooved ring rolls. The expansion is controlled by the degree of interference or engagement between the grooves of the rolls. FIG. 19 shows an expanded and apertured FT935 film. The open area is generally greater than 5% and preferably between 5 and 40%. The enlargement of the apertures is created by the thinning of the bridges **8** connecting the apertures **6.** The result is a film with thick regions (first region **2**) and thin regions (second region **4**) across the cross direction of the film created by molecular deformation of the film, The second region **4** contains the apertures 6 and the bridge **8.** No interaperture bridges **10** are formed when a single slit aperture is first formed from a tooth.

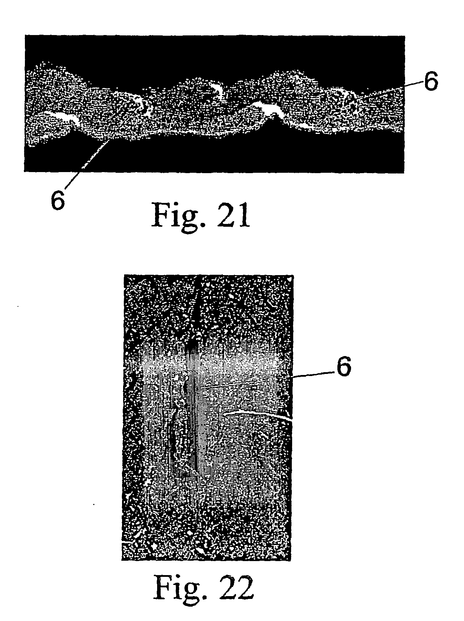

FIGS. 20-23 illustrate the same film at different steps of processing and in different views. An alternative embodiment is fractured or apertured film that is created using cutting teeth such as triangular knife teeth versus rectangular teeth. This tooth design is more forgiving of the film properties. FIG. 20 shows an apertured film having slit apertures created using the knife teeth. This apertured film is also suitable for abrasive applications. FIG. 21 shows the same film as shown in FIG. 20 in a side view. This view of the apertured film illustrates the three dimensional deformation. The deformation is more rounded or humped shaped with a slit or fracture at the peak of the deformation. FIG. 22 shows a magnified view of the aperture of FIG. 20 where the fractured surface is exemplified by rough and outward facing lips. The combination of three dimensionality and outward facing lips provides uses in abrasive applications such as scrubbing or exfoliation. The open area of these films is typically between 0 and 5%. This apertured film can be created at high production speeds.

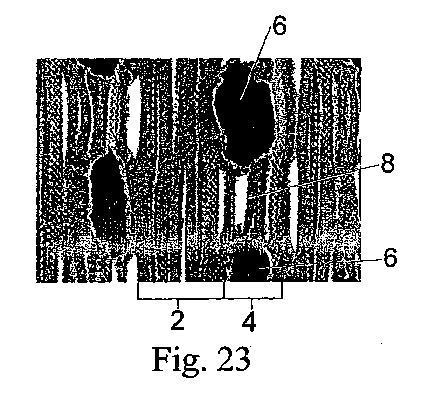

The apertured or fractured film of FIGS. 20-22 can be expanded using the grooved set of ring rolls for a second processing step. The resulting structure is an expanded apertured film wherein the deformations are flattened out and the apertures expanded. FIG. 23 shows this expanded apertured film. The open area is generally greater than 5% and preferably between 5 and 40%.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A method for making an apertured web (1), the method comprising the steps of:
a. providing a polymer film web;
b. providing a deformation means (108) comprising intermeshing rolls comprising a first roll (112) and a second roll (110), each roll rotating about an axis (A₁), the axes being parallel in the same plane, wherein said first roll comprises a plurality of ridges (115) and corresponding groves which extend unbroken about the entire circumference of the first roll, and wherein said second roll comprises a plurality of rows of circumferentially spaced teeth (114) that extend in spaced relationship about a portion of said second roll; and
c. deforming said polymer film web via said deformation means to form a deformed web comprising plurality of tent-like structures (12).

2. The method according to Claim 1, further comprising a stretching means to stretch said deformed web.

3. The method according to Claim 2 wherein said stretching means comprises an incremental stretching means (132) to stretch said deformed web.

4. The method according to Claim 2 wherein said stretching step is in the same step as said deforming step.

5. The method according to Claim 1 wherein the deformation means comprises square teeth.

6. The method according to any one of the preceding claims, wherein said first roll (112) and said second roll (110) intermesh such that said teeth (114) of said second roll extend into said grooves of said first roll.

## Patentansprüche

1. Verfahren zum Herstellen einer perforierten Bahn (1), wobei das Verfahren die folgenden Schritte umfasst:
a. Bereitstellen einer Polymerfolienbahn,
b. Bereitstellen eines Verformungsmittels (108), umfassend ineinander greifende Walzen, umfassend eine erste Walze (112) und eine zweite Walze (110), wobei sich jede Walze um eine Achse (A1) dreht, wobei die Achsen in derselben Ebene parallel sind, wobei die erste Walze mehrere Erhebungen (115) und entsprechende Rillen umfasst, die ununterbrochen um den gesamten Umfang der ersten Walze verlaufen, und wobei die zweite Walze mehrere Reihen von im Umfang beabstandeten Zähnen (114) umfasst, die in beabstandeter Beziehung um einen Abschnitt der zweiten Walze verlaufen, und
c. Verformen der Polymerfolienbahn mit dem Verformungsmittel, um eine verformte Bahn zu bilden, die mehrere zeltartige Strukturen (12) umfasst.

2. Verfahren nach Anspruch 1, das ferner ein Dehnungsmittel zum Dehnen der verformten Bahn umfasst.

3. Verfahren nach Anspruch 2, wobei das Dehnungsmittel ein inkrementelles Dehnungsmittel (132) zum Dehnen der verformten Bahn umfasst.

4. Verfahren nach Anspruch 2, wobei der Dehnungsschritt in dem gleichen Schritt erfolgt wie der Verformungsschritt.

5. Verfahren nach Anspruch 1, wobei das Verformungsmittel quadratische Zähne umfasst.

6. Verfahren nach einem der vorstehenden Ansprüche, wobei die erste Walze (112) und die zweite Walze (110) so ineinander greifen, dass die Zähne (114) der zweiten Walze in die Rillen der ersten Walze ragen.

## Revendications

1. Procédé pour fabriquer une nappe perforée (1), le procédé comprenant les étapes consistant à :
a. fournir une nappe de film polymère ;
b. fournir un moyen de déformation (108) comprenant des rouleaux qui s'engrènent comprenant un premier rouleau (112) et un deuxième rouleau (110), chaque rouleau tournant autour d'un axe (A1), les axes étant parallèles dans le même plan, dans lequel ledit premier rouleau comprend une pluralité de crêtes (115) et de rainures correspondantes qui s'étendent sans interruption sur environ toute la circonférence du premier rouleau, et dans lequel ledit deuxième rouleau comprend une pluralité de rangées de dents (114) espacées sur la circonférence qui s'étendent en relation espacée autour d'une partie dudit deuxième rouleau ; et
c. déformer ladite nappe de film polymère par l'intermédiaire dudit moyen de déformation de façon à former une nappe déformée comprenant une pluralité de structures de type tente (12).

2. Procédé selon la revendication 1, comprenant en outre un moyen d'étirage pour étirer ladite nappe déformée.

3. Procédé selon la revendication 2, dans lequel ledit moyen d'étirage comprend un moyen d'étirage incrémentiel (132) pour étirer ladite nappe déformée.

4. Procédé selon la revendication 2, dans lequel ladite étape d'étirage est dans la même étape que ladite étape de déformation.

5. Procédé selon la revendication 1, dans lequel le moyen de déformation comprend des dents carrées.

6. Procédé selon l'une quelconque des revendications précédentes, dans lequel ledit premier rouleau (112) et ledit deuxième rouleau (110) s'engrènent de telle sorte que lesdites dents (114) dudit deuxième rouleau s'étendent dans lesdites rainures dudit premier rouleau.
